# EUROPEAN PATENT APPLICATION

(11) **EP 4 471 076 A1**
(43) Date of publication of application: **04.12.2024**
(21) Application number: 23746768.3
(22) Date of filing: 18.01.2023
(51) Int. Cl.: C08G 65/20

(54) **COPOLYMERIZATION REACTION PRODUCT OF TETRAHYDROFURAN AND PLANT-DERIVED 2-METHYLTETRAHYDROFURAN, AND METHOD FOR PRODUCING SAME**

(30) Priority: 28.01.2022 JP 2022011623
(71) Applicant: Hodogaya Chemical Co., Ltd., Tokyo, 105-0021 (JP)
(72) Inventor: MATSUMOTO, Akihiro, Tokyo 105-0021 (JP); HAMADA, Tatsuya, Tokyo 105-0021 (JP); AOKI,Yoshikazu, Tokyo 105-0021 (JP)
(74) Representative: Wächtershäuser & Hartz Patentanwaltspartnerschaft mbB
(86) International application number: PCT/JP2023/001271
(87) International publication number: WO 2023/145554

(57) **Abstract**

2MeTHF is expected to be used to develop poly(tetramethylene ether) glycol as a bio-urethane material, which has new performance with excellent chemical and mechanical properties. The purpose is to provide poly(tetramethylene ether) glycol that does not cause coloration and its production method so that its value as an industrial raw material is not reduced.

Poly(tetramethylene ether) glycol which is a copolymerization reaction product of tetrahydrofuran and plant-derived 2-methyltetrahydrofuran, wherein the 2-methyltetrahydrofuran is 2-methyltetrahydrofuran purified by a purification step that includes contacting the 2-methyltetrahydrofuran with an acid.

## Description

### Field of the Invention

The present invention relates to poly(tetramethylene ether) glycol, which is a copolymerization reaction product of tetrahydrofuran and plant-derived 2-methyltetrahydrofuran, and a method for producing the same.

### Background Art

Poly(tetramethylene ether) glycol is one of the polyols used in polyurethane materials, polyester materials, and polycarbonate materials. Polyurethane and polyester materials are used in applications such as elastomers and spandex. Among poly(tetramethylene ether) glycols, poly(tetramethylene ether) glycol obtained by copolymerization of tetrahydrofuran (hereinafter also referred to as THF) and alkyltetrahydrofuran is known to have excellent softness and elongation properties when used as a raw material for spandex (Patent Documents 1 and 2).

In fibers used for spandex, poly(tetramethylene ether) glycol with coloration (poor hue) decreases its value as an industrial raw material from a design and design standpoint. For this reason, purification methods for THFs, the raw material for poly(tetramethylene ether) glycol, have been proposed in the past. Examples of the purification methods include, focusing on dihydrofuran as an impurity, a method of converting dihydrofuran to a high-boiling point product by reacting it with an acid catalyst or acids to facilitate separation by distillation (Patent Documents 2 to 4), and a method of purifying THF by converting an impurity to be purified, which is peroxide, to γ-butyrolactone (Patent Document 5). In these reports, methods of producing the THFs to be purified are not limited, and no distinction is made between petroleum-based, bio-based, and by-product-based THFs, and THFs with substituents are also included in the purification targets. However, even though many reports have been made, a purification method limited to 2-methyltetrahydrofuran (hereinafter also referred to as 2MeTHF) has not yet been found.

Dihydrofuran is also mentioned as an impurity in THFs (especially THF and 3-methyltetrahydrofuran) used in the production of poly(tetramethylene ether) glycol (Patent Documents 2 to 4). Again, there are no reports focusing on impurities in 2MeTHF.

On the other hand, in recent years, there has been a social demand for the development of non-petroleum products using biomass-derived raw materials for the purpose of realizing a recycling-oriented society and countering the depletion of fossil fuels. Currently, biopolyols made from vegetable oils exist, but there are a limited number of biopolyols that are best suited for spandex fiber applications. Biopolyether polyols and biopolyurethane resins with excellent properties are in demand (Patent Document 6).

2MeTHF is generally produced by the furfural or levulinic acid method starting from biomass-derived materials. Therefore, 2MeTHF is expected to be a raw material for biopolyether polyol. However, biopolyether polyols synthesized from 2MeTHF had the problem of coloration. A method for producing poly(tetramethylene ether) glycol that does not cause coloration is desired so that its value as an industrial raw material is not reduced.

### PRIOR ART REFERENCE

### Patent Document

Patent Document 1: Japanese Unexamined Patent Publication No. 2011-162780
Patent Document 2: Japanese Patent No. 4144656
Patent Document 3: Japanese Patent No. 6191213
Patent Document 4: Japanese Patent No. 6601006
Patent Document 5: Japanese Unexamined Patent Publication No. 2016-17041
Patent Document 6: WO 2018/220983

### Summary of the Invention

### Problems to be Solved by the Invention

The use of plant-derived 2MeTHF as a bio-urethane material is expected to develop new poly(tetramethylene ether) glycols with excellent chemical and mechanical properties. The problem to be solved by this invention is to provide poly(tetramethylene ether) glycol that does not cause coloration and a method for producing the same so that its value as an industrial raw material is not reduced.

### Means for Solving the Problems

As a result of diligently studying the aforementioned problem, the inventors have found that poly(tetramethylene ether) glycol with reduced coloration can be produced by using, in the copolymerization reaction of tetrahydrofuran and 2-methyltetrahydrofuran, 2-methyltetrahydrofuran that has been purified in a purification step that includes a step of contacting it with an acid as 2-methyltetrahydrofuran. In other words, the present invention is summarized as follows.
[1] Poly(tetramethylene ether) glycol which is a copolymerization reaction product of tetrahydrofuran and plant-derived 2-methyltetrahydrofuran, with Hazen color scale (APHA color) of 60 or less.
[2] Poly(tetramethylene ether) glycol as described in [1], wherein the Hazen color scale (APHA color) is less than 30.
[3] Poly(tetramethylene ether) glycol as described in [1], wherein a biobased carbon content (%) of the plant-derived 2-methyltetrahydrofuran is 100%.
[4] Poly(tetramethylene ether) glycol as described in [1], wherein the tetrahydrofuran is plantderived and a biobased carbon content (%) of the tetrahydrofuran is 97.00 to 100%.
[5] Poly(tetramethylene ether) glycol as described in [1], having a number average molecular weight of 500 to 5000.
[6] A method for producing poly(tetramethylene ether) glycol, comprising:
   obtaining purified plant-derived 2-methyltetrahydrofuran by a purification step including contacting plant-derived 2-methyltetrahydrofuran with an acid; and
   copolymerizing the purified plant-derived 2-methyltetrahydrofuran and tetrahydrofuran.
[7] The method for producing poly(tetramethylene ether) glycol as described in [6], wherein a purity of the purified plant-derived 2-methyltetrahydrofuran is 99.90% or more.
[8] The method for producing poly(tetramethylene ether) glycol as described in [6], wherein the acid used in the purification step is a mineral acid or a strongly acidic ion exchange resin.
[9] The method for producing poly(tetramethylene ether) glycol as described in [8], wherein in the purification step, the plant-derived 2-methyltetrahydrofuran and the acid are brought into contact in a temperature range of 20 to 80°C.
[10] The method for producing poly(tetramethylene ether) glycol as described in [8], wherein in the purification step, the plant-derived 2-methyltetrahydrofuran and the acid are brought into contact in a temperature range of 30 to 60°C.
[11] The method for producing poly(tetramethylene ether) glycol as described in [8], wherein the purification step comprises:
   bringing plant-derived 2-methyltetrahydrofuran into contact with a mineral acid or a strongly acidic ion exchange resin;
   separating 2-methyltetrahydrofuran and the mineral acid or the strongly acidic ion exchange resin and dehydrating; and
   extracting purified 2-methyltetrahydrofuran by distillation.
[12] The method for producing poly(tetramethylene ether) glycol as described in [6], wherein a purity of the tetrahydrofuran is 99.50% or more.
[13] The method for producing poly(tetramethylene ether) glycol as described in [6], wherein in the copolymerization step, a ratio (by mass) of tetrahydrofuran to plant-derived 2-methyltetrahydrofuran is 99:1 to 50:50.
[14] The method for producing poly(tetramethylene ether) glycol as described in [6], wherein in the copolymerization step, a strong acid catalyst is added in an amount of 5 to 40% by mass relative to the total mass of tetrahydrofuran and 2-methyltetrahydrofuran.
[15] The method for producing poly(tetramethylene ether) glycol as described in [6], wherein in the copolymerization step, a strong acid catalyst is added in an amount of 8 to 40% by mass relative to the total mass of tetrahydrofuran and 2-methyltetrahydrofuran.

### Effects of the Invention

According to the present invention, the coloration of poly(tetramethylene ether) glycol obtained by copolymerization reaction of THF and 2MeTHF can be suppressed, and its industrial value for applications such as spandex fiber is not compromised. In addition, it will be possible to provide biopolyurethane materials using the poly(tetramethylene ether) glycol.

### MODE FOR CARRYING OUT THE INVENTION

The following is a detailed description of the embodiments of the present invention. The following description is an example of an embodiment of the invention, and the invention is not specified to these contents, but can be implemented with various variations within the scope of the gist.

### [Biobased carbon-containing poly(tetramethylene ether) glycol with reduced coloration]

One aspect of the invention is a biobased carbon-containing poly(tetramethylene ether) glycol with reduced coloration. More specifically, it is poly(tetramethylene ether) glycol which is a copolymerization reaction product of tetrahydrofuran and plant-derived 2-methyltetrahydrofuran, with Hazen color scale (APHA color) of 60 or less, preferably 30 or less.

The Hazen color scale (APHA color) is a measure of the degree of coloration of a liquid chemical product at room temperature or a chemical product that becomes molten when heated, and the color of a solution containing 1mg of platinum in the form of hexachloroplatinum ion and 2mg of cobalt(II) chloride hexahydrate in 1 liter is scale 1 [See JIS K0071-1 Test methods for colour of chemical products- Part 1: Estimation of colour in Hazen units (platinum-cobalt colour scale), JIS K0071-1 Annex A (preparation of platinum-cobalt color standard solution)].

Biobased carbon means carbon derived from biomass feedstock, and in the present invention, at least 2-methyltetrahydrofuran as a feedstock is derived from plants. Plant-derived 2-methyltetrahydrofuran is produced by the furfural and levulinic acid methods starting from plant-derived raw materials, as described in the production methods described below, but can be used without limitation to those methods.

Biobased carbon content (%) is known as a measure of the degree of plant origin (ASTM D6866 standard). Biobased carbon content (%) means the percentage (%) of the carbon mass of the biomass-derived component of the total carbon mass in the product. Carbon mass derived from natural polymers such as starch powder and plant fiber is also included. The preferred biobased carbon content of plant-derived 2-methyltetrahydrofuran is 97.00 to 100%, more preferably 100%.

As plant-derived 2-methyltetrahydrofuran which is a raw material for biobased carbon-containing poly(tetramethylene ether) glycol with reduced coloration which is an aspect of the present invention, by using plant-derived 2-methyltetrahydrofuran purified by the purification method described in the production of poly(tetramethylene ether) glycol described below, a specified low Hazen color scale (APHA color) can be achieved.

The other raw material, tetrahydrofuran, includes specifically, and without limitation, petroleum-based, bio-based (plant-derived (biomass-derived)), byproduct-based, and other tetrahydrofurans. In the present invention, it is preferred that the tetrahydrofuran be of plant-derived. When the tetrahydrofuran (THF) to be used is produced from plant-derived (or biomass-derived) raw materials, it may be produced by known methods such as the furfural method. The purity of the tetrahydrofuran is preferably 99.00 to 100%, more preferably 99.50 to 100%, and even more preferably 99.70 to 100%. The biobased carbon content (%) of tetrahydrofuran to be used is preferably 97.00 to 100%, more preferably 98.00 to 100%, and most preferably 100%.

Purity, as used herein, is the purity determined by gas chromatography. More specifically, the purity is calculated based on the percent area of the chromatogram.

The number average molecular weight of biobased carbon-containing poly(tetramethylene ether) glycol with reduced coloration, which is an aspect of the present invention, is preferably 500 to 5000. When the number average molecular weight (Mn) of poly(tetramethylene ether) glycol is less than 500, the polyurethane resin polymerized and produced using the poly(tetramethylene ether) glycol may become harder, and the rubber modulus and tensile strength of the polyurethane resin may decrease. If the number average molecular weight is more than 5000, the elongation of the polyurethane resin may become too large and its properties as a resin may be compromised. Therefore, the number average molecular weight (Mn) of the biobased carbon-containing poly(tetramethylene ether) glycol with reduced coloration, which is an aspect of the present invention, is preferably 500 to 5000, more preferably 1000 or more, more preferably 4500 or less, even more preferably 1500 or more, and even more preferably 4000 or less.

With regard to other items, the description in the method for producing poly(tetramethylene ether) glycol, described below, can be used as a reference.

### [Method for producing poly(tetramethylene ether) glycol]

Another aspect of the present invention is a production method capable of producing biobased carbon-containing poly(tetramethylene ether) glycol with reduced coloration described above.

2MeTHF to be used in the production method is generally produced by the furfural or levulinic acid method starting from plant-derived raw materials (wherein as used herein, products generally described as "biomass-derived" are also described as "plant-derived"), but is not limited to 2MeTHF produced by that method. 2MeTHF starting from plant-derived raw materials is preferred to maximize the main purpose of the invention. Furfural can be synthesized from plant-derived biomass materials such as polysaccharides, i.e., cellulose and hemicellulose, and lignin, which can be recovered from agricultural waste such as corn cores and sugarcane pomace, and is a compound that can be synthesized in an environmentally friendly process.

As used herein, when referring to "biobutanediol," "biotetrahydrofuran," "biopolyol," "biopolyether polyol," etc., the terms refer to plant-derived low molecular weight or high molecular weight compounds that have properties equivalent to those of petroleum-based products. As used herein, when referring to "bio-polyurethane materials," the term refers to those in which at least 50% by mass or more of the raw materials thereof are components derived from plant-derived compounds.

In the following, the method for producing biobased carbon-containing "poly(tetramethylene ether) glycol" with reduced coloration, which is an aspect of the present invention, is described in detail. The "poly(tetramethylene ether) glycol" of the present invention is a copolymerization reaction product of the "tetrahydrofuran" and the "plant-derived 2-methyltetrahydrofuran", The method includes "purification step" and "copolymerization reaction step" mentioned below.

### <Purification step>

The method for producing poly(tetramethylene ether) glycol includes a purification step of purifying unpurified 2MeTHF, which is a step of contacting the "plant-derived 2-methyltetrahydrofuran" (unpurified 2MeTHF) with an acid. More specifically, purified 2MeTHF is obtained by conducting a purification step including contacting the "plant-derived 2-methyltetrahydrofuran" (unpurified 2MeTHF) with an acid followed by distillation.

The purity of the product can be increased to 99.90% or more by the purification step. It is preferred that the purity of the "plant-derived 2-methyltetrahydrofuran" (unrefined 2MeTHF) to be used in the purification step is as high as possible, for example, 99.50% or more.

The step of purifying 2MeTHF carried out in the method for producing poly(tetramethylene ether) glycol according to the present invention preferably consists of the following steps.

Purification step (I): Bringing 2MeTHF containing 2-methylfuran into contact with a mineral acid (typically sulfuric acid, nitric acid, hydrochloric acid) or a strongly acidic ion exchange resin.

Purification step (II): Separating 2MeTHF and the mineral acid or the strongly acidic ion exchange resin and dehydrating.

Purification step (III): Extracting 2MeTHF by distillation.

The details of each step are described in turn below.

Sulfuric acid, nitric acid, and hydrochloric acid can be used as the mineral acid used in the purification step (I) of bringing 2MeTHF into contact with the mineral acid or the strongly acidic ion exchange resin. The concentration of the mineral acid used is not particularly limited, and is preferably 10 to 80% by mass for sulfuric acid, 5 to 45% by mass for nitric acid, 5 to 30% by mass for hydrochloric acid. Sulfuric acid of 40 to 70% by mass is more preferred. The amount of mineral acid to be contacted is preferably 5 to 100% by mass and more preferably 5 to 30% by mass relative to the amount of 2MeTHF.

Strongly acidic ion exchange resins in the purification step (I) include, specifically, aromatic sulfonic acid type cation exchange resins, benzyl sulfonic acid type cation exchange resins and the like. The matrix resins for these exchange resins include styrene, methacrylic, phenolic resins and the like. Specific commercial products include the DIAION SK series, DIAION PK series (both manufactured by Mitsubishi Chemical Corporation) and the like. The amount of strongly acidic ion exchange resin to be contacted is preferably 5 to 100% by mass relative to the amount of 2MeTHF.

In the purification step (I), the temperature at which 2MeTHF is contacted with the acid can be selected arbitrarily in the range of 78°C (the boiling point of 2MeTHF), for example, from 20°C to 80°C. A temperature between 30°C and 60°C is preferred to increase contact efficiency. The contact method can be either batch or continuous. When performed in a batch system, mixing and stirring is performed for 0.1 to 10 hours, preferably 0.5 to 3 hours. When performed in a continuous system, it is operated so that the contact time with the mineral acid or the strongly acidic ion exchange resin is from 0.1 to 10 hours, preferably from 0.5 to 3 hours. The mineral acid or the strongly acidic ion exchange resin after use can also be used when the purification step is repeated.

The step of separating 2MeTHF and the mineral acid or the strongly acidic ion exchange resin in the purification step (II) is performed after stopping mixing and stirring and allowing the mixture to stand still, followed by liquid separation in the case of a batch system. Separation by filtration can also be performed if a strongly acidic ion exchange resin is used. In the case of a continuous system, the separating step is done in standing and liquid separation facilities or filtration facility following a contact column or kettle with a mineral acid or strongly acidic ion exchange resin. The temperature at the time of liquid separation is not limited, and the step can be simplified by using the same temperature as the contact temperature with the mineral acid or the strongly acidic ion exchange resin. The time to be required for standing and liquid separation varies depending on the type and concentration of the mineral acid used. It is desirable to set a settling time that is longer than the time for the interface between the 2MeTHF layer and the mineral acid layer to be recognized, as this will avoid step losses. After separation of the mineral acid, a further operation may be performed to remove the mineral acid layer remaining in the 2MeTHF layer by adding 10 to 100% by mass water to 2MeTHF, mixing and stirring, and allowing the mixture to stand and separate.

The dehydration step in the purification step (II) can be performed by using alkali metal hydroxides or alkaline earth metal hydroxides. Alkali metal hydroxide or alkaline earth metal hydroxide is added to the 2MeTHF layer after the mineral acid layer is separated off, and the isolated aqueous layer is separated and removed. Specific examples of alkali metal hydroxides include sodium, potassium, lithium, and cesium hydroxides, with sodium hydroxide being preferred. Specific examples of alkaline earth metal hydroxides include beryllium, magnesium, calcium, and barium hydroxides. The dehydration step can be omitted if the use of a strongly acidic ion exchange resin results in little water contamination and there is no need for dehydration. Dehydration facilities can be either batch or continuous.

In the purification step (III), 2MeTHF is extracted by distillation. The distillation method can be either batch or continuous. Distillation is performed using a distillation column with a theoretical number of stages between 1 and 50. The theoretical number of stages in a distillation column may be 5 or more to improve the purification efficiency of the distillate mixture, and preferably 10 or more. The theoretical number of stages in a distillation column is preferably 50 or less to avoid consuming more energy than necessary for distillation.

The lower limit of the reflux ratio of the distillation column is 0.01 or more, preferably 0.1 or more, and the upper limit thereof is 10 or less, preferably 5 or less. If the lower limit is too small, the distillate mixture may not be sufficiently separated. If the upper limit is too large, the energy required for distillation may become too great, and economic efficiency may be reduced.

The pressure in the distillation of 2MeTHF can be from atmospheric to reduced pressure. The degree of pressure reduction, when performed under reduced pressure, can be set arbitrarily between 1 kPa and 101 kPa. Reducing the degree of pressure reduction decreases the energy required for evaporation, but increases the energy required to lower the temperature of the cooling medium used in the cooling coagulator to coagulate the vapor. Therefore, it is preferable to set energy-efficient degree of pressure reduction in light of the equipment used. The distillation temperature is determined based on the evaporation temperature of 2MeTHF, which is determined by the pressure setting.

When distillation is carried out under ambient pressure in a batch system, in the initial stage of refining, an azeotropic composition of THF and water, which has a lower boiling point than 2MeTHF, and a low-boiling fraction are distilled out. Therefore, 0 to 10% by mass of the liquid after the purification step (II) treatment supplied to distillation is removed as first distillate. Purified 2MeTHF is then distilled off. The percentage of purified 2MeTHF extracted is 80 to 95% by mass of the liquid after the purification step (II) treatment supplied to distillation. Finally, 2MeTHF containing many high-boiling components, corresponding to 0 to 10% by mass of the liquid after the purification step (II) treatment supplied to distillation" remains in the distiller. Each of the above steps (I) through (III) can also be operated in a continuous system.

The mineral acid water discharged in the purification step (II) and the first distillate separated in the purification step (III) can be used to recover the remaining 2MeTHF in them in a separate distillation operation. The purification method according to the present invention can also be applied to the recovered 2MeTHF.

The purified 2MeTHF of high purity obtained in the above purification steps (I) to (III) is suitable for use as a raw material for the production of the poly(tetramethylene ether) glycol of the present invention, especially for the production (polymerization reaction) of "poly(tetramethylene ether) glycol with Hazen color scale (APHA color) of 60 or less". It is also possible to use a mixture of THF and/or 3-methyltetrahydrofuran with the aforementioned 2MeTHF as a raw material for the production (polymerization reaction) of poly(tetramethylene ether) glycol (copolymerization reaction step). Therefore, the "method for producing poly(tetramethylene ether) glycol" according to the present invention includes the "method for producing poly(tetramethylene ether) glycol" by the following "copolymerization reaction step" using purified 2-methyltetrahydrofuran (purified 2MeTHF) mentioned above.

### <Copolymerization reaction step>

In the production method according to the present invention, specifically in the copolymerization reaction step of tetrahydrofuran (THF) and plant-derived 2-methyltetrahydrofuran (2MeTHF), the ratio of THF to 2MeTHF (mass ratio) can be selected arbitrarily in consideration of the required performance and cost (specifically, the cost of the copolymerization reaction step, reaction time, percent yield, actual yield, physical properties of the resulting copolymerization reactants or poly(tetramethylene ether) glycol, physical properties of the polyurethane resin obtained using the poly(tetramethylene ether) glycol, and other related performance and costs). However, if the ratio of 2MeTHF is extremely low, the expected performance cannot be achieved, and conversely, if the ratio of 2MeTHF is too high, it is cost-prohibitive due to reduced reactivity. Therefore, the ratio of THF to 2MeTHF (mass ratio) is preferably 99:1 to 50:50, more preferably 97:3 to 55:45, and particularly preferably 93:7 to 60:40.

In the production method according to the present invention, the copolymerization reaction of THF and 2MeTHF is preferably carried out by adding a strong acid catalyst in an amount of 5 to 40% by mass relative to the total mass of THF and 2MeTHF. The lower limit of the amount of such strong acid catalyst to be added is preferably 5% by mass, more preferably 8% by mass, and particularly preferably 15% by mass from the viewpoint of good reaction conversion rate and good percent yield. The upper limit of the amount of the strong acid catalyst to be added is preferably 40% by mass, and more preferably 36% by mass from the viewpoint of avoiding a decrease in molecular weight at reaction equilibrium due to an increase in the number of reaction active species.

In the production method according to the present invention, the copolymerization reaction of THF and 2MeTHF is performed by mixing THF and 2MeTHF at the preferred mass ratio of THF to 2MeTHF described above and feeding a strong acid catalyst while maintaining a temperature of 0 to 50°C.

In the production method according to the present invention, specific examples of catalysts used for the copolymerization reaction of THF and 2MeTHF include acetic anhydride, perchloric acid, fluorosulfonic acid, and fuming sulfuric acid. Strong acid catalysts may be used alone, or combinations of several strong acids can also be used. If two or more strong acids are used, each of the two or more strong acids may be used in several stages, each with a temperature range of 0 to 50°C.

Poly(tetramethylene ether) glycol is obtained by hydrolyzing the reaction solution of the polymerization reaction described above. Hydrolysis is preferably carried out by adding water in an amount of 10 to 100% by mass relative to the mass of the reaction solution at a temperature of 30 to 120°C. The aqueous layer is separated from the reaction solution after completion of hydrolysis, and unreacted raw materials, etc. are separated to obtain poly(tetramethylene ether) glycol.

The poly(tetramethylene ether) glycol obtained in the copolymerization reaction step of THF and 2MeTHF in the production method of the present invention, preferably has a number average molecular weight (Mn) of 500 to 5000, as described in the section on the biobased carbon-containing poly(tetramethylene ether) glycol with reduced coloration.

The poly(tetramethylene ether) glycol of the present invention, i.e., the poly(tetramethylene ether) glycol obtained by the production method according to the present invention, can be used to produce polyurethane materials also using polyisocyanate and chain extenders as main raw materials.

In the chemical reaction in the production step for polyurethane materials described above, as the polyisocyanate compounds, polyisocyanate compounds having two or more isocyanate groups in a molecule are used. Examples thereof include polyisocyanates such as tolylene diisocyanate (TDI), diphenylmethane diisocyanate (MDI), xylylene diisocyanate (XDI), isophorone diisocyanate (IPDI), hexamethylene diisocyanate (HDI), naphthalene diisocyanate (NDI), and hydrogenated diphenylmethane diisocyanate. These may be used alone or in a mixture of two or more.

In the chemical reaction of the production step for polyurethane materials described above, as a chain extender, chain extenders with two or more hydroxyl groups and amino groups are suitably used. Examples thereof include ethylene glycol, 1,3-propanediol, 1,4-butanediol, 1,5-pentanediol, 1,6-hexanediol, glycerin, trimethylolpropane, ethylenediamine, propylenediamine, phenylenediamine, diaminodiphenylmethane and the like. These may be used alone or in a mixture of two or more.

In the chemical reaction of the production step for polyurethane materials, it is possible to produce plant-derived polyurethane (biopolyurethane) resin materials by using biomass-derived raw materials, specifically the plant-derived 2MeTHF, plant-derived THF (biotetrahydrofuran), or plant-derived butanediol (biobutanediol), in combination as starting materials. Bio-polyurethane materials have a biomass-derived component content of 50 to 80% by weight relative to 100% by weight of polyurethane material. In general, the higher the content of plant-derived (biomass-derived) components in the resin, the more environmentally friendly it is. In this regard, however, to achieve bio-polyurethane materials having a biomass-derived component content of 80% by mass or more, raw materials such as polyisocyanate compounds and chain extenders, in addition to 2MeTHF, must also be plant-derived, which currently presents many technical and economic challenges.

For the rest, the above section on the biobased carbon-containing poly(tetramethylene ether) glycol with reduced coloration can be used as a guide.

### Examples

Hereinbelow, the present invention will be described in detail by means of examples. The invention is not limited to the following examples, but can be implemented with various variations within the scope of the gist of the invention. In the purification and synthesis examples, unless otherwise specified, reagents, etc. manufactured by KISHIDA CHEMICAL CO. LTD., ORGANO CORPORATION, Tokyo Chemical Industry Co., Ltd., FUJIFILM Wako Pure Chemical Corporation, and other known chemical manufacturers or distributors were used.

The analytical instruments and sample analysis methods used in the examples are as follows.

### [Analysis of 2MeTHF]

### Gas chromatography (GC):

Equipment: Gas chromatograph manufactured by SHIMADZU CORPORATION, model: GC-2014
Analysis column: DB-1 by T&D Corporation (I.D. 0.25 mm x 30 m, film thickness 0.25 µm)
Column temperature: 40°C (6.5 min.) → (20°C /min.) → 200°C (15 min.)
Flow rate: He, 0.7 mL/min.
Shot volume: 0.1 µL
Inj. temperature: 200°C
Detector: FID
Det. temperature: 200°C
RANGE: 10 ¹

### [Coloration Judgment]

The degree of coloration was determined by comparing the Hazen color scale (APHA color) by the test method described in the Japanese Industrial Standard JIS K0071-1:2007.

### [Determination of number average molecular weight (Mn) of poly(tetramethylene ether) glycol]

The hydroxyl value of poly(tetramethylene ether) glycol was measured by the test method described in the Japanese Industrial Standard JIS K1557-1:2007, and Mn was calculated.

### [Purification Example 1 (Purified Example 1), Unpurification Example 1 (Unpurified Example 1) and Unpurification Example 2 (Unpurified Example 2)]

### Purification 1 of 2MeTHF

In a 3L reaction vessel, 1000g of unpurifed 2MeTHF (Unpurified Example 1) (Tokyo Chemical Industry Co., Ltd.) and 100g of 60% sulfuric acid (KISHIDA CHEMICAL CO., LTD.) were charged and the mixture was stirred at 60°C for 1 hour. After standing the mixture for 30 minutes, the liquid was separated to obtain 700 g of the oil layer. The oil layer was washed with 300 g of water and the liquid was separated to obtain 650 g of the washed oil layer.

In a 2L vessel, 400 g of the 60% sulfuric acid aqueous layer and 350 g of the washed water layer, which were separated as described above, were charged, and the mixture was heated to 90°C, and 250 g of 2MeTHF was recovered by single distillation.

650 g of the washed oil layer described above and 250 g of the recovered 2MeTHF described above were mixed, and 50 g of sodium hydroxide (KISHIDA CHEMICAL CO., LTD.) was added thereto and the mixture was stirred for 30 minutes. Decantation gave 800 g of crude 2MeTHF (Unpurified Example 2). The purity (%) measured by gas chromatography (hereafter referred to as GC purity) of unpurified 2MeTHF (Unpurified Example 1) and the crude 2MeTHF obtained (Unpurified Example 2) are shown in Table 1.

800 g of the crude 2MeTHF obtained above was charged to a 1L vessel and heated to 80°C. Distillation was performed using a distillation column with 10 theoretical stages. At a reflux ratio of 1, 40 g of the initial distillate was distilled and removed. At a reflux ratio of 0.1, purified 2MeTHF 700 g (70% yield relative to unpurified 2MeTHF) was obtained. The purity (%) (GC purity) of the purified 2MeTHF (Purified Example 1) obtained is shown in Table 1.

The biobased carbon content (%) of 2MeTHF used in Purification Example 1 was confirmed to be 100%.

### (Measurement method: ASTM D6866-22 Method B, Chikyu Kagaku Kenkyusho)

### [Purification Example 2 (Purified Example 2)] Purification 2 of 2MeTHF

Purified 2MeTHF 720 g (72% yield (relative to unpurified 2MeTHF)) was obtained in the same manner as in Purification Example 1, except that 40 g of the first distillate in Purification Example 1 was replaced by 20 g. The GC purity of the purified 2MeTHF obtained (Purified Example 2) is shown in Table 1.

### [Purification Example 3 (Purified Example 3)] Purification 3 of 2MeTHF

Purified 2MeTHF 720 g (65% yield (relative to unpurified 2MeTHF)) was obtained in the same manner as in Purification Example 1, except that the sulfuric acid concentration in Purification Example 1 was changed to 25%. The GC purity of the purified 2MeTHF obtained (Purified Example 3) is shown in Table 1.

**[Table 1]**

| | 2MeTHF | GC purity (%) |
|---|---|---|
| Unpurification Example 1 (Unpurified Example 1) | Unpurified 2MeTHF | 99.87 |
| Unpurification Example 2 Unpurified Example 2 | Crude 2MeTHF | 99.88 |
| Purification Example 1 (Purified Example 1) | Purified 2MeTHF | 99.95 |
| Purification Example 2 (Purified Example 2) | Purified 2MeTHF | 99.90 |
| (Purification Example 3) Purified Example 3 | Purified 2MeTHF | 99.90 |

### [Example 1] Synthesis 1 of THF-2MeTHF copolymerization reactant

In a 1L volume reaction vessel, 255 g of THF (KISHIDA CHEMICAL CO., LTD.) and 45 g of purified 2MeTHF obtained in Purification Example 1 were charged and the mixture was cooled at 0°C. 2g of 70% perchloric acid (KISHIDA CHEMICAL CO., LTD.) was added to the reaction vessel. Next, 58 g of 25% fuming sulfuric acid (FUJIFILM Wako Pure Chemical Corporation) was added dropwise at -5 to 5°C in 5 hours. After stirring the reaction solution at -5 to 5°C for 2 hours, 160 g of water was added thereto, and the mixture was heated to 70°C and refluxed for 3 hours. After refluxing, the solution was allowed to stand at 65 to 70°C for 1 hour, and then the aqueous layer was removed. In the oil layer, 100 g of water and 100 g of THF were added, and the mixture was heated to 70°C, and refluxed for 3 hours. After refluxing, the solution was allowed to stand at 65 to 70°C for 1 hour, and then the aqueous layer was removed. 200 mL of ion exchange resin IRA-96S (ORGANO CORPORATION) was added to the oil layer and the mixture was stirred for 1 hour at room temperature (around 20 to 25°C). After filtration to remove the ion exchange resin, the solution was concentrated under reduced pressure at 120°C to obtain THF-2MeTHF copolymerization reactant (125 g, 42% yield). The results of the above synthesis (mass, and mass ratio of each raw material, etc., and yield) and the analysis results of the obtained THF-2MeTHF copolymerization reactant (number average molecular weight (Mn) and Hazen color scale (APHA color)) are shown in Table 2.

### [Example 2] Synthesis 2 of THF-2MeTHF copolymerization reactant

In a 1L volume reaction vessel, 360 g of THF (KISHIDA CHEMICAL CO., LTD.) and 240 g of purified 2MeTHF obtained in Purification Example 2 were charged and the mixture was cooled at 0°C. 8g of 70% perchloric acid (KISHIDA CHEMICAL CO., LTD.) was added to the reaction vessel. Next, 205 g of 25% fuming sulfuric acid (FUJIFILM Wako Pure Chemical Corporation) was added dropwise at -5 to 5°C in 5 hours. After stirring the reaction solution at -5 to 5°C for 5 hours, 100 g of water was added, and the mixture was heated to 70°C and refluxed for 3 hours. After refluxing, the solution was allowed to stand at 65 to 70°C for 1 hour, and then the aqueous layer was removed. In the oil layer, 100 g of water and 100 g of THF were added, and the mixture was heated to 70°C, and refluxed for 3 hours. After refluxing, the solution was allowed to stand at 65 to 70°C for 1 hour, and then the aqueous layer was removed. 200 mL of ion exchange resin IRA-96S (ORGANO CORPORATION) was added to the oil layer and the mixture was stirred for 1 hour at room temperature (around 20 to 25°C). After filtration to remove the ion exchange resin, the solution was concentrated under reduced pressure at 120°C to obtain THF-2MeTHF copolymerization reactant (200 g, 30% yield). The results of the above synthesis and the analysis results of the obtained THF-2MeTHF copolymerization reactant are shown in Table 2 as in Example 1.

### [Examples 4, 5, 7, 9 to 13] Syntheses 4, 5, 7, 9 to 13 of THF-2MeTHF copolymerization reactants

The syntheses were performed in the same manner as in Example 1, except that the amounts of THF, 2MeTHF, 70% perchloric acid, and fuming sulfuric acid were changed to the values in Table 2, to obtain THF-2MeTHF copolymerization reactants. As in Example 1, the results are shown in Table 2.

### [Examples 3, 6 and 8] Syntheses 3, 6 and 8 of THF-2MeTHF copolymerization reactants

The syntheses were performed in the same manner as in Example 1, except that in Example 1, purified 2MeTHF obtained in Purification Example 3 was used instead of Purification Example 1, and the amounts of THF, 2MeTHF, 70% perchloric acid and fuming sulfuric acid were changed to the values in Table 2, to obtain THF-2MeTHF copolymerization reactants. As in Example 1, the results are shown in Table 2.

### [Comparison Example 1] Synthesis 14 of THF-2MeTHF copolymerization reactant

In a 1L volume reaction vessel, 360 g of THF and 240 g of unpurified 2MeTHF, which was Unpurified Example 1, were charged and the mixture was cooled at 0°C. 8g of 70% perchloric acid was added to the reaction vessel. Next, 205 g of 25% fuming sulfuric acid was added dropwise at -5 to 5°C for 5 hours. After stirring the reaction solution at -5 to 5°C for 5 hours, 100 g of water was added, and the mixture was heated to 70°C and refluxed for 3 hours. After refluxing, the solution was allowed to stand at 65 to 70°C for 1 hour, and then the aqueous layer was removed. In the oil layer, 100 g of water and 100 g of THF were added, and the mixture was heated to 70°C, and refluxed for 3 hours. After refluxing, the solution was allowed to stand at 65 to 70°C for 1 hour, and then the aqueous layer was removed. 200 mL of ion exchange resin IRA-96S (ORGANO CORPORATION) was added to the oil layer and the mixture was stirred for 1 hour at room temperature (around 20 to 25°C). After filtration to remove the ion exchange resin, the solution was concentrated under reduced pressure at 120°C to obtain THF-2MeTHF copolymerization reactant (200 g, 30% yield). The results of the above synthesis and the analysis results of the obtained THF-2MeTHF copolymerization reactant are shown in Table 2 as in Example 1.

### [Comparison Example 2] Synthesis 15 of THF-2MeTHF copolymerization reactant

In a 1L volume reaction vessel, 360 g of THF and 240 g of crude 2MeTHF, which was Unpurified Example 2, were charged and the mixture was cooled at 0°C. 8g of 70% perchloric acid was added to the reaction vessel. Next, 205 g of 25% fuming sulfuric acid was added dropwise at -5 to 5°C for 5 hours. After stirring the reaction solution at -5 to 5°C for 5 hours, 100 g of water was added, and the mixture was heated to 70°C and refluxed for 3 hours. After refluxing, the solution was allowed to stand at 65 to 70°C for 1 hour, and then the aqueous layer was removed. In the oil layer, 100 g of water and 100 g of THF were added, and the mixture was heated to 70°C, and refluxed for 3 hours. After refluxing, the solution was allowed to stand at 65 to 70°C for 1 hour, and then the aqueous layer was removed. 200 mL of ion exchange resin IRA-96S (ORGANO CORPORATION) was added to the oil layer and the mixture was stirred for 1 hour at room temperature (around 20 to 25°C). After filtration to remove the ion exchange resin, the solution was concentrated under reduced pressure at 120°C to obtain THF-2MeTHF copolymerization reactant (200 g, 30% yield). The results of the above synthesis and the analysis results of the obtained THF-2MeTHF copolymerization reactant are shown in Table 2 as in Example 1.

### [Comparison Example 3] Synthesis 16 of THF-2MeTHF copolymerization reactant

Unpurified 2MeTHF (Unpurified Example 1) was distilled using a distillation column with 10 theoretical stages, omitting the acid wash with 60% sulfuric acid and the dehydration step with sodium hydroxide in Purification Example 1. The synthesis was performed in the same manner as in Example 1, except that this 2MeTHF was used and the amounts of THF, 2MeTHF, 70% perchloric acid, and fuming sulfuric acid were changed to the values in Table 2, to obtain THF-2MeTHF copolymerization reactant. As in Example 1, the results are shown in Table 2.

**[Table 2]**

| | THF (g) | 2MeTHF (g) | THF:2MeTHF (100 in total) | 70% Perchloric acid (g) | Fuming nitric acid (g) | Yield (%) | Molecular weight | Hazen color scale |
|---|---|---|---|---|---|---|---|---|
| Example 1 | 255 | 45 | 85:15 | 2 | 58 | 42 | 2200 | 10 |
| Example 2 | 360 | 240 | 60:40 | 8 | 205 | 30 | 3200 | 10 |
| Example 3 | 225 | 75 | 75:25 | 2 | 68 | 29 | 1442 | 30 |
| Example 4 | 2340 | 1260 | 65:35 | 220 | 690 | 43 | 1500 | 10 |
| Example 5 | 5580 | 990 | 85:15 | 40 | 1260 | 38 | 1600 | 10 |
| Example 6 | 225 | 75 | 75:25 | 3 | 68 | 34 | 1731 | 30 |
| Example 7 | 5980 | 590 | 91:9 | 40 | 1050 | 34 | 1780 | 10 |
| Example 8 | 225 | 75 | 75:25 | 3 | 68 | 36 | 2423 | 50 |
| Example 9 | 6960 | 690 | 91:9 | 150 | 1840 | 26 | 2920 | 10 |
| Example 10 | 3250 | 1580 | 67:33 | 90 | 1080 | 28 | 3200 | 10 |
| Example 11 | 6500 | 1150 | 85:15 | 150 | 1840 | 26 | 3450 | 10 |
| Example 12 | 7370 | 730 | 91:9 | 160 | 1670 | 19 | 3680 | 10 |
| Example 13 | 6890 | 1220 | 85:15 | 160 | 1950 | 19 | 3920 | 10 |
| Comparative Example 1 | 360 | 240 | 60:40 | 8 | 205 | 30 | 3100 | 80 |
| Comparative Example 2 | 360 | 240 | 60:40 | 8 | 205 | 30 | 2900 | 70 |
| Comparative Example 3 | 98 | 42 | 70:30 | 3 | 29 | 35 | 4349 | 75 |

Table 2 shows that each poly(tetramethylene ether) glycol, which is a copolymerization reaction product of THF and purified 2MeTHF of the present invention, has a lower Hazen color scale (APHA color), and is poly(tetramethylene ether) glycol with less coloration compared to each poly(tetramethylene ether) glycol synthesized with unpurified 2MeTHF in Comparative Examples.

### Industrial applicability

The method for producing poly(tetramethylene ether) glycol according to the present invention can provide poly(tetramethylene ether) glycol without coloration. The poly(tetramethylene ether) glycol obtained by the production method of the present invention is a plant-derived, and high quality poly(tetramethylene ether) glycol with little coloration, and is an excellent bio-urethane material for a recycling-based society.

## Claims

1. Poly(tetramethylene ether) glycol which is a copolymerization reaction product of tetrahydrofuran and plant-derived 2-methyltetrahydrofuran, with Hazen color scale (APHA color) of 60 or less.

2. Poly(tetramethylene ether) glycol according to claim 1, wherein the Hazen color scale (APHA color) is less than 30.

3. Poly(tetramethylene ether) glycol according to claim 1, wherein a biobased carbon content (%) of the plant-derived 2-methyltetrahydrofuran is 100%.

4. Poly(tetramethylene ether) glycol according to claim 1, wherein the tetrahydrofuran is plant-derived and a biobased carbon content (%) of the tetrahydrofuran is 97.00 to 100%.

5. Poly(tetramethylene ether) glycol according to claim 1, having a number average molecular weight of 500 to 5000.

6. A method for producing poly(tetramethylene ether) glycol, comprising:
obtaining purified plant-derived 2-methyltetrahydrofuran by a purification step including contacting plant-derived 2-methyltetrahydrofuran with an acid; and
copolymerizing the purified plant-derived 2-methyltetrahydrofuran and tetrahydrofuran.

7. The method for producing poly(tetramethylene ether) glycol according to claim 6, wherein a purity of the purified plant-derived 2-methyltetrahydrofuran is 99.90% or more.

8. The method for producing poly(tetramethylene ether) glycol according to claim 6, wherein the acid used in the purification step is a mineral acid or a strongly acidic ion exchange resin.

9. The method for producing poly(tetramethylene ether) glycol according to claim 8, wherein in the purification step, the plant-derived 2-methyltetrahydrofuran and the acid are brought into contact in a temperature range of 20 to 80°C.

10. The method for producing poly(tetramethylene ether) glycol according to claim 8, wherein in the purification step, the plant-derived 2-methyltetrahydrofuran and the acid are brought into contact in a temperature range of 30 to 60°C.

11. The method for producing poly(tetramethylene ether) glycol according to claim 8, wherein the purification step comprises:
bringing plant-derived 2-methyltetrahydrofuran into contact with a mineral acid or a strongly acidic ion exchange resin;
separating 2-methyltetrahydrofuran and the mineral acid or the strongly acidic ion exchange resin and dehydrating; and
extracting purified 2-methyltetrahydrofuran by distillation.

12. The method for producing poly(tetramethylene ether) glycol according to claim 6, wherein a purity of the tetrahydrofuran is 99.50% or more.

13. The method for producing poly(tetramethylene ether) glycol according to claim 6, wherein in the copolymerization step, a ratio (by mass) of tetrahydrofuran to plant-derived 2-methyltetrahydrofuran is 99:1 to 50:50.

14. The method for producing poly(tetramethylene ether) glycol according to claim 6, wherein in the copolymerization step, a strong acid catalyst is added in an amount of 5 to 40% by mass relative to the total mass of tetrahydrofuran and 2-methyltetrahydrofuran.

15. The method for producing poly(tetramethylene ether) glycol according to claim 6, wherein in the copolymerization step, a strong acid catalyst is added in an amount of 8 to 40% by mass relative to the total mass of tetrahydrofuran and 2-methyltetrahydrofuran.
